(19) Europäisches Patentamt
European Patent Office
Office européen des brevets

(11) Publication number: **0 002 127**
**A1**

(12) # EUROPEAN PATENT APPLICATION

(21) Application number: **78300627.3**

(22) Date of filing: **14.11.78**

(51) Int. Cl.[2]: **A 61 K 7/15**

(30) Priority: **15.11.77 GB 47490/77**
**11.05.78 GB 47490/77**

(43) Date of publication of application: **30.05.79**
**Bulletin 79/11**

(84) Designated Contracting States: **CH DE FR NL**

(71) Applicant: **Moss, Arthur, 50 Tolmers Road, Cuffley Hertfordshire (GB)**

(72) Inventor: **Moss, Arthur, 50 Tolmers Road, Cuffley Hertfordshire (GB)**

(74) Representative: **Warden, John C. et al, R.G.C. JENKINS & CO. 53/64 Chancery Lane, London WC2A 1AY (GB)**

(54) **Shaving composition.**

(57) A shaving composition incorporating a betaine. The preferred composition is a transparent gel based on hydroxyethyl cellulose and also incorporating a quaternary ammonium compound and a soluble oil obtained from plummage grease.

EP 0 002 127 A1

## FIELD OF THE INVENTION

The present invention relates to a shaving composition, that is to say a composition which is applied to the face in order to soften the hair preparatory to application of a razor.

In the past there have been available for this purpose soaps in the form of sticks or bowls adapted to be applied to the face as a lather with a brush. There have also been available creams adapted to be applied with a wet brush for the same purpose, called "lather creams". There have also been available so called "brushless shaving creams" which are applied simply by hand. All of these compositions had little more effect or stated purpose than the mere wetting of the hair. The compositions applied with a brush are generally time consuming although more effective than the brushless creams, which suffer from the additional disadvantage that they are immiscible with water, resulting in clogging of the razor and difficulty in subsequent cleaning of the basin.

SUMMARY OF THE INVENTION

I have now found that surprising benefits can be obtained by the incorporation in a shaving composition of a betaine. The betaine should be of a cosmetically acceptable type, many examples of which are known and have been used for example in shampoos for ten years or more. Other uses to which betaines have been applied are soaps and detergents in general and fabric softeners. They are well known for their anti-static properties and many of them have, to a greater or lesser extent, useful anti-irritant properties, which accounts for their use particularly in shampoos.

The particular properties which render them beneficial in shaving compositions are not clearly known. The use of an agent having anti-static properties, while of obvious benefit in relation to the washing of hair of substantial length, would not appear prima facie to have a clear application to the treatment of stubble. It is believed however, that the anti-static properties may contribute to some extent but that the particular action of the betaines on the roots of the hair provides the surprising ability for close

shaving which is achieved through use of the invention. The invention is not however limited to the operation of any particular mechanism.

The amount of betaine to be employed in the composition may in general vary from 0.1 to 10 per cent by weight, depending on the type of end product. For example 0.5 per cent is preferred for gel formulations to be described and 3% for cream formulations.

Any of the betaines advocated for use in personal toilet soaps or shampoos are in general suitable. Many such compounds are disclosed, e.g. in the following British patent specifications: 995,353; 1,084,739; 1,087,414; 1,087,415; 1,274,005; 1,291,424; 1,355,233.

The word "betaine" is used herein to mean any zwitterionic quaternary ammonium compound having positive and negative charges which are internally neutralised. Thus the expression includes compounds sometimes called sulphobetaines or sultaines. "Quaternary ammonium compound" as used herein excludes internally neutralised compounds.

DETAILED DESCRIPTION OF EMBODIMENTS

The preferred betaines may be represented

by the following formula:

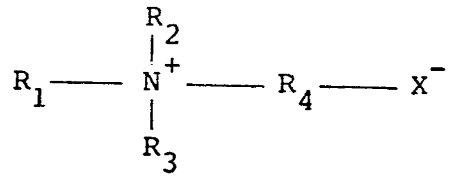

where $R_1$ is alkyl or alkenyl of 8-20 carbon atoms which may be saturated or unsaturated, may be interrupted by hetero-atoms or groups such as -O-, -COO or CONH and may have one or more hydroxy substituents, $R_2$ and $R_3$ are alkyl residues of 1 to 4 carbon atoms, $R_4$ is an alkylene residue of 1 to 3 carbon atoms and X is $-CO\bar{O}$, $-S\bar{O}_3$, or $-\bar{O}SO_3$.

$R_1$ may also include 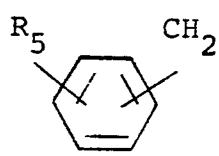 - groups as described in British patent specifications Nos. 1,274,005 and 1,291,424, although this is not preferred. $R_5$ is alkyl of 8 to 24, preferably 10 to 18 carbon atoms.

Preferably $R_1$ has 12 to 18 carbon atoms and $R_2$ and $R_3$ are methyl.

The preferred betaines are those described in British patent specification No. 1,084,739, i.e. those in the formula above in which $R_1$ represents a hydrophobic group which is an alkyl or alkenyl group having from 8 - 18 carbon atoms, for example a dodecyl, tridecyl,

myristyl, cetyl, stearyl, 2-mythylpentadecyl group or an alkyl phenyl group possessing an alkyl substituent having from 6 to 16 carbon atoms, for example a p-octylphenyl, p-nonylphenyl or p-dodecylphenyl group. Preferably $R_1$ represents a lauryl and/or myristyl group, which may be derived from an alcohol which has been obtained by reduction of the mixture of fatty acids or their derivatives obtained from coconut oil and may be a whole cut, middle-cut or narrow-cut distillate fraction; "middle-cut coconut alcohol" comprises at least 50 per cent by weight of dodecyl alcohol and "narrow-cut" coconut oil at least 70 per cent by weight.

$R_2$ and $R_3$ are preferably methyl but may be ethyl or 2-hydroxyethyl or 2-hydroxypropyl.

Preferably $R_4$ is methylene but alternatives are ethylene, 2-hydroxyethylene and 2-hydroxypropylene.

The group $X^-$ is preferably a carboxy group.

The betaine employed in the preferred embodiment to be described is that sold under the trade mark EMPIGEN BB which can be represented by the formula above in which

$R_2$ and $R_3$ are methyl, $R_4$ is methylene, $X^-$ is carboxyl and $R_1$ is predominantly lauryl/myristyl ($C_{12}/C_{14}$). This is normally sold as a 30 per cent by weight solution in deionised water, containing 0.5 per cent of free amine and having a pH of 8. It has extremely mild detergent action and is non-irritant.

The following are alternative betaines within the general formula above:

lauryl dimethyl carboxymethyl betaine; lauryl dimethyl alpha-carboxyethyl betaine; cetyl dimethyl carboxymethyl betaine; lauryl bis-(2-hydroxyethyl) carboxymethyl betaine; lauryl bis-(2-hydroxyethyl) betasulphatoethyl betaine, lauryl dimethyl beta-sulphatoethyl betaine; stearyl bis-(2-hydroxypropyl) carboxymethyl betaine; cetyl bis-(2-hydroxyethyl) beta-sulphatopropyl betaine; olelyl dimethyl gamma-carboxypropyl betaine; and lauryl bis-(2-hydroxypropyl) alpha-carbosyethyl betaine, 1-myristyl dimethylammonio) propane - 3 - sulphonate and 1- (myristyl - dimethylammonio) - 2 - hydroxypropane - 3 - sulphonate and the coconut oil-derived alkyl dimethyl betaine and the alkyl amidopropyl dimethyl betaine detergents

in which the alkyl group contains 3% $C_8$, 7% $C_{10}$, 48% $C_{12}$, 18% $C_{14}$, 9% $C_{16}$ and 10% $C_{18}$.

The composition of the invention preferably also includes a proportion of a quaternary ammonium compound which should be of the type which is cosmetically acceptable e.g. in soaps, creams and the like and which have anti-microbial properties. The quaternary ammonium compound is utilised to stabilise the composition against deterioration. However it is also useful for its detergent properties and softening effect upon the hair, and for its antistatic properties. Many suitable quaternary ammonium compounds are known and are used in shampoos and soaps, e.g. in the earlier British patents above disclosed, especially 1,355,233 and also 1,029,043. The preferred compound is cetyl trimethyl-ammonium bromide which is sold under the trade mark Cetrimide. The quaternary ammonium compound may be employed in amount by weight of 0.05 to 5 per cent of the composition, preferably about 2 per cent.

The composition may be in the form of a shaving soap, a lather cream, or a brushless cream, in its broad aspect. However, I have

developed, in accordance with an important preferred aspect of the invention, a novel composition in the form of a substantially transparent gel. This gel is especially effective for the following reasons.

It can be applied in small quantities, e.g. about 1ml to the moistened face directly with the hand. It provides a lubricant to the razor, giving a smooth action, and it allows the stubble to be viewed during the shaving operation, which is impossible with lathers and creams. It is not normally necessary to shave more than once but the gel can in any case be redistributed by a second gentle rub with the hand if a second shave is necessary. The whole operation is extremely rapid and the face left in a smooth and soft condition.

The preferred gel can be based upon colloidal dispersing agents or gelling agents such as hydroxyethyl cellulose. The preferred gelling agents are those sold under the trade mark Tylose H. As sold, these are odourless and neutral. They have a cellulose ether content of about 93%, about 5% moisture (as supplied) about 2% residual sodium acetate, about 2%

molar substitution, about 35% average $-OC_2H_4$ content and a specific gravity of 1.4. Thermal decomposition begins at about 200°C. The preferred ingredient Tylose H 4000 has an average molecular weight of 190,000 and a viscosity (Hoeppler viscosity of 2 per cent solution at 20°C) of 3000 to 5000 cP.

The Tylose is preferably employed in dilute aqueous solution, e.g. 2.5% by weight. This solution may constitute about 95% by weight of the composition and may vary e.g. between 80 to 98 per cent. The solution concentration may vary from e.g. 1 to 4% by weight.

Preferably there is also included in the gel a small quantity e.g. about 2.5% by weight or generally from 1 to 3 per cent of an oil. The preferred oil is that sold under the trade make Pur-Cellin Soluble. This is a soluble form of oil derived from the plummage grease of water fowl. Chemically the base oil is an alkyl branched fatty acid ester with a low solidification point (around 0°C). This material is absorbed by the skin, leaving it soft and moist.

The invention is hereafter further described with reference to certain non-limitation examples.

Example 1

The following example gives a preferred formulation of shaving gel in accordance with the invention.

| | Per Cent by Weight |
|---|---|
| Cetrimide | 0.5 |
| Empigen BB (30% by weight aqueous solution) | 2 |
| Pur-Cellin Soluble | 2.5 |
| Tylose H 4000 (2.5% solution) | 95 |

Example 2

In Example 1, the quaternary ammonium compound Cetrimide is replaced by the same proportion of Dehyquart SP (Trade Mark). This is an oxyethyl alkyl ammonium phosphate commonly used in hair conditioners.

Example 3

The following is an example of a brushless shaving cream.

| | Per Cent by Weight |
|---|---|
| Cetyl stearyl alcohol | 15% |
| Empigen BB (30% solution) | 10% |
| Glycerol or sorbitol | 15% |
| Pur-Cellin Oil | 5% |
| Perfumes (as desired) | |
| Water | balance |

In general the cetyl stearyl alcohol can be replaced by any suitable emulsifying wax including commercial brands of self-emulsifying wax within the broad limits of 1 to 15%. The betaine can be any suitable betaine as already described within the broad limits described, but a larger minimum amount will be necessary where non-self-emulsifying waxes are used. The glycerol or sorbitol can be very broadly between 5 to 20%. The Pur-Cellin oil can be replaced by Pur-Cellin Soluble and either of these can vary from 1 to 10%. Furthermore, a quaternary ammonium compound may be added to replace part of the betaine, but preferably not more than half the betaine by weight, should be so replaced.

Example 4

In order to prepare a lather shaving cream, the receipe of Example 4 can be used with the replacement of the glycerol or sorbitol by coconut oil-diethanolamide.

In order to prepare a solid shaving soap, a conventional toilet soap formulation may be used containing up to 10% by weight of betaine. Part of the betaine may be replaced by a quaternary ammonium compound but the total of the mixture should preferably not exceed 10% by weight, and there is preferably not less than 1% and not more than 5% of such a quaternary ammonium compound.

78300627.3
0002127

CLAIMS:

1. A shaving composition incorporating a cosmetically acceptable betaine.

2. A shaving composition as claimed in claim 1 wherein the amount of betaine is from 0.1 to 10% by weight of total weight.

3. A shaving composition as claimed in claim 1 or claim 2 wherein the betaine is represented by the formula:

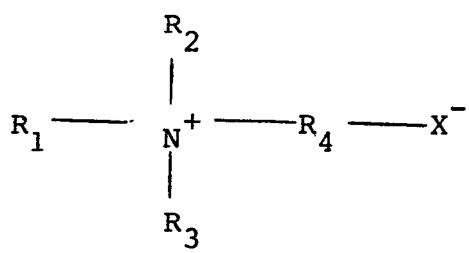

where $R_1$ is selected from

(a) alkyl and alkenyl of 8-20 carbon atoms which may be saturated or unsaturated, may be interrupted by hetero-atoms or groups such as -O-, -COO or CONH and may have one or more hydroxy substituents, and

(b) 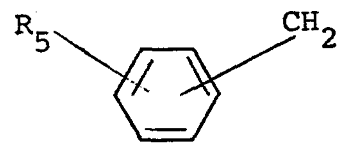

where $R_5$ is alkyl of 8 to 24 carbon atoms and where $R_2$ and $R_3$ are alkyl residues of 1 to 4 carbon atoms, $R_4$ is an alkylene residue of 1 to 3 carbon atoms and X is $-CO\bar{O}$, $-SO_3^-$, or $-OSO_3^-$.

4. A shaving composition as claimed in claim 3 wherein $R_1$ is selected from dodecyl, tridecyl, myristyl, cetyl, stearyl, a 2-mythylpentadecyl group and an alkyl phenyl group possessing an alkyl substituent having from 6 to 16 carbon atoms,

wherein $R_2$ and $R_3$ are selected from methyl, ethyl, 2-hydroxyethyl, and 2-hydroxypropyl,

wherein $R_4$ is selected from methylene, ethylene, 2-hydroxyethylene and 2-hydroxypropylene, and

wherein the group $X^-$ is a carboxy group.

5. A shaving composition as claimed in claim 3 and in which $R_2$ and $R_3$ are methyl, $R_4$ is methylene, $X^-$ is carboxyl and $R_1$ is predominantly lauryl and myristyl.

6. A shaving composition as claimed in any preceding claim incorporating a cosmetically acceptable quaternary ammonium compound in an amount by weight of 0.05 to 5% of the composition.

7. A shaving composition as claimed in claim 6 wherein the quaternary ammonium compound comprises cetyl trimethyl ammonium bromide.

8. A shaving composition as claimed in any preceding claim in the form of a substantially transparent gel.

9. A shaving composition as claimed in claim 8 incorporating as a gelling agent, hydroxyethyl cellulose which has (prior to incorporation) an average molecular weight of 190,000 and a viscosity (Hoeppler viscosity of 2% solution at 20$^{o}$C) of 3000 to 5000 cP.

10. A shaving composition as claimed in claim 8 or claim 9 wherein the gel contains hydroxyethyl cellulose in the form of an aqueous solution of from 1 to 4% by weight constituting from 80 to 98% of the gel.

11. A shaving composition as claimed in claim 15 wherein the gel includes from 1 to 3% of a soluble form of a base oil derived from the plummage grease of water fowl and consisting of an alkyl branched fatty acid ester.

European Patent Office

# EUROPEAN SEARCH REPORT

Application number
EP 78 30 0627

## DOCUMENTS CONSIDERED TO BE RELEVANT

| Category | Citation of document with indication, where appropriate, of relevant passages | Relevant to claim |
|---|---|---|
| X | FR - A - 2 229 392 (WILKINSON SWORD)<br>* Page 4, line 18 - page 5, line 19; page 6, lines 19-21; page 9, lines 14-17; example 19 *<br>-- | 1-5 |
| X | GB - A - 1 479 706 (WILKINSON SWORD)<br>* Page 1, lines 9-12; page 3, lines 26-65; page 4, lines 70-78; page 4, lines 110-116; example 7; claims *<br>-- | 1-5 |
| | FR - A - 2 208 976 (MODOKEMI)<br>* Page 1, lines 1-34; page 5, lines 25,26 *<br>-- | 1-5 |
| | FR - A - 2 010 816 (COLGATE-PALMOLIVE)<br>* Page 5, line 22 - page 6, line 14; page 8, line 23 - page 9, line 30 *<br>-- | 1-7 |
| | US - A - 2 833 693 (NAIMARK)<br>* Column 1, line 10 - column 4, line 50 *<br>---- | 8-10 |

CLASSIFICATION OF THE APPLICATION (Int. Cl.²)

A 61 K 7/15

TECHNICAL FIELDS SEARCHED (Int.Cl.²)

A 61 K 7/15
C 11 D 1/90
1/92

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 09-02-1979 | JONAS |